# EUROPEAN PATENT SPECIFICATION

(11) **EP 3 448 438 B1**
(45) Date of publication and mention of the grant of the patent: **17.06.2020**
(21) Application number: 17721577.9
(22) Date of filing: 25.04.2017
(51) Int. Cl.: A61L 2/18, A61L 2/20, B01D 1/00

(54) **A HYDROGEN PEROXIDE EVAPORATION DEVICE, AND A METHOD FOR EVAPORATING HYDROGEN PEROXIDE**
WASSERSTOFFPEROXIDVERDAMPFUNGSVORRICHTUNG UND VERFAHREN ZUR VERDAMPFUNG WASSERSTOFFPEROXID
DISPOSITIF D'ÉVAPORATION DE PEROXYDE D'HYDROGÈNE, ET PROCÉDÉ POUR L'ÉVAPORATION DE PEROXYDE D'HYDROGÈNE

(30) Priority: 27.04.2016 SE 1650566
(43) Date of publication of application: 06.03.2019
(73) Proprietor: Tetra Laval Holdings & Finance S.A., 1009 Pully (CH)
(72) Inventor: RUNNBERG, Bo, 273 98 Smedstorp (SE); NILSSON, Peter Jo, 24545 Staffanstorp (SE)
(74) Representative: Tetra Pak - Patent Attorneys SE
(86) International application number: PCT/EP2017/059822
(87) International publication number: WO 2017/186733

(56) References cited:
- WO-A1-2012/167857
- FR-A1- 2 852 514
- US-A- 3 119 004
- US-A- 4 896 478
- US-A1- 2005 095 168
- US-B1- 6 339 678

## Description

### Technical field

The present disclosure relates to manufacturing of packages such as carton based packages for liquid food, and in particular to a hydrogen peroxide evaporation device for providing a sterilization agent during such manufacturing.

### Background

It is commonly known to use a carton based packaging material to form product containers, such as containers for enclosing and storing liquid food.

In order to ensure the required quality of the final package, e.g. in terms of food safety and integrity, the packaging material may comprise different layers. As an example, a packaging material may comprise a core material layer with at least one decorative layer applied on one side thereof making up the outer surface of the final package, and a polymeric composition or layer on the opposite or inner side. The polymeric composition may in some cases be provided with a protective film such as aluminum; the polymeric composition thus normally also includes an outer, or distal layer being in contact with the product intended to be contained in the final package.

Typically the packaging material is formed into semi-finished packages before they are filled with its desired content. Especially for food content it is required to sterilize the material of the package prior to filling. For such sterilization it is common to spray a gas mixture of hydrogen peroxide and air into the semi-finished package before any final content is introduced. The hot gas mixture will condense at the inner surface of the semi-finished package to form a thin liquid layer. This thin layer of sterilizing agent is then exposed to UV light for killing any microorganisms present inside the semi-finished package, and finally the remaining hydrogen peroxide will be vented before filling and sealing of the package is performed.

For providing the hot gas mixture of hydrogen peroxide it is required to feed a liquid solution of hydrogen peroxide and water through an evaporator. Due to heat exposure the mix of hydrogen peroxide and water will evaporate, whereby the gaseous solution is forwarded to a spray nozzle configured to discharge the gaseous sterilizing agent into the ready-to-fill packages. As there is normally a required minimum temperature for the hydrogen peroxide gas entering the packages for sterilization, a number of considerations must be made. First of all, it is desired to have a relatively small-sized evaporator and secondly the desired temperature should be reaches as fast as possible. These two prerequisites suggest that the liquid hydrogen peroxide should be fed through a very hot evaporator. However, using too high temperatures for the evaporator will create a potential risk that the materials of the evaporator, in particular stainless steel, lose their corrosion resistance. Further, the rate of decomposition or breakdown of hydrogen peroxide will rapidly increase with not only increased temperatures, but also for any corrosion present. As of today there is no solution for a hydrogen peroxide evaporator which provides the desired heating of the gas within the required time frame and which ensures no corrosion of the evaporator materials.

In view of this, it would be desired to have an improved hydrogen peroxide evaporator device in order to at least partly overcoming the disadvantages of prior art solutions.

### Summary

An object of the present disclosure is to solve the above-mentioned problems.

According to a first aspect, an evaporation device for evaporating hydrogen peroxide is provided. The device comprises a housing body having at least two fluid channels arranged therein, which fluid channels are connected to each other to form a common fluid line between an inlet and an outlet, and at least one heating element positioned within said housing body for heating said fluid channels. A first fluid channel, being directly connected to the fluid inlet, is positioned relative the at least one heating element such that its inner walls will be heated to a first temperature, and a second fluid

Example prior art solutions in the field of peroxide sterilization are shown in the patent documents US2005/095168 A1, FR2852514 A1, US4896478 A and US6339678 B1.

US2005/095168 A1 discloses a method and an apparatus for generating vapor in conjunction with sterilization of rooms, buildings, medical devices, bottles and packages. The apparatus comprises a heating block which is inductively heated and which comprises electrically non-conductive and an electromagnetically responsive material which define an interior passage. Steam, usually water vapor together with hydrogen peroxide is passed through this interior passages and becomes heated and vaporized when it comes into contact with the walls of the interior passage. The vapor flowing out of the interior passage is then used for sterilization. The temperature over the length of the interior passage is more or less constant.

FR2852514 A1 deals with an apparatus for generating a vaporized form of an aqueous hydrogen peroxide solution for delivery to an accumulator chamber. The accumulator chamber is in contact with a sterilization chamber and the vaporized sterilant in the form of hydrogen peroxide solution is periodically released into the sterilization chamber thus ensuring a constant concentration of the vaporized sterilant, while at the same time the accumulator is recharged at predefined intervals as well. The temperature in the accumulator inlet is higher than in the steam accumulator outlet.

US4896478 A discloses an apparatus and a method for sterilization of packaging container by means of a liquid sterilizing agent which is vaporized by being passed through a spray pipe. In order to achieve good vaporization capacity a rotary flow movement along the central axis of the spray pipe is achieved by means of air swirl bodies and coil spring inserts. Liquid which not yes has been vaporized is directed opposite the direction of the main stream of the sterilizing agent and made to stay longer on the pipe walls of the spray pipe.

Finally, US6339678 B1, describes a somewhat similar solution to the one discussed in US4896478, where a sterilizing agent is guided through heating channels and first heated to a first temperature whereafter it is superheated to a second higher temperature. The sterilizing agent is firstly introduced into heating channels heating it to a first temperature below which film boiling of the sterilizing agent begins. After the first temperature is registered by a sensor heating to a second temperature is controlled, which heats the sterilizing agent in a counter flow directed in the opposite direction to the flow of vapor. In this way, the first temperature is kept substantially constant.

channel, being directly connected to the fluid outlet, is positioned relative the at least one heating element such that its inner walls will be heated to a second temperature, said second temperature being higher than the first temperature.

In an example the housing body is a solid block and the fluid channels are channels provided inside said block. The housing body may be made of Aluminum or stainless steel, making it particularly suitable for applications involving hydrogen peroxide.

In an example said at least one heating element extends along a longitudinal axis of said housing body, whereby efficient heating of the liquid to be evaporated is accomplished. The at least one heating element may e.g. be an electrical heating element.

The first temperature may be selected such that liquid hydrogen peroxide entering the first fluid channel will be entirely evaporated while flowing through the first fluid channel. For optimal heat transfer, the first temperature is preferably 30°C above the boiling temperature of the liquid to be evaporated.

The first temperature may e.g. be between 120-140°C, and the second temperature may e.g. be between 200-250°C.

In an example each fluid channel extends from a first end face of the housing body to an opposite end of the housing body, and each end face of the housing body is closed by means of a respective end plate. At least one fluid channel may for such example be connected to an adjacent fluid channel by means of a fluid connection formed as a groove in one of said end faces. Manufacturing of the device is thus greatly improved.

Said at least one groove may be closed by means of one of said end plates.

According to a second aspect, a method for evaporating hydrogen peroxide is provided. The method comprises feeding a liquid aqueous solution of hydrogen peroxide through a first fluid channel arranged in a housing body, and subsequently through a second fluid channel also arranged within said housing body, which fluid channels are connected to each other to form a common fluid line between an inlet and an outlet. The method also comprises heating the inner walls of said fluid channels by means of at least one heating element arranged within said housing body, whereby the first fluid channel, being directly connected to the fluid inlet, is positioned relative the at least one heating element such that its inner walls will be heated to a first temperature, and the second fluid channel, being directly connected to the fluid outlet, is positioned relative the at least one heating element such that its inner walls will be heated to a second temperature, said second temperature being higher than the first temperature.

The first temperature is preferably approximately 30°C above the boiling temperature of the liquid to be evaporated, and the second temperature may be between 200-250°C.

In an example the concentration of the liquid aqueous solution of hydrogen peroxide is between 2-5%. Moreover, the first temperature may be selected such that liquid aqueous solution of hydrogen peroxide entering the first fluid channel will be entirely evaporated while flowing through the first fluid channel.

### Short description of the drawings

Fig. 1 illustrates an exemplary form, fill and seal packaging machine that includes a system for hydrogen peroxide treatment embodying the principles of the present disclosure.
Fig. 2 is a schematic view of a hydrogen peroxide evaporator according to prior art.
Fig. 3a is a cross-sectional isometric view of a hydrogen peroxide evaporation device according to an example.
Fig. 3b is a cross-sectional front view of the hydrogen peroxide evaporation device shown in Fig. 3a.
Fig. 4a is a cross-sectional front view of a hydrogen peroxide evaporation device accordign to a further example.
Fig. 4b is a cross-sectional isomteric view of a hydrogen peroxide evaporation device according to a yet further example.
Fig. 4c is a cross-sectional isomteric view of a hydrogen peroxide evaporation device according to a yet further example.
Fig. 5 is a schematic view of a method according to an example.

### Detailed description

While the present disclosure is susceptible of example in various forms, there is shown in the drawings and will hereinafter be described presently preferred examples with the understanding that the present disclosure is to be considered an exemplification of the disclosure and is not intended to limit the disclosure to the specific examples illustrated.

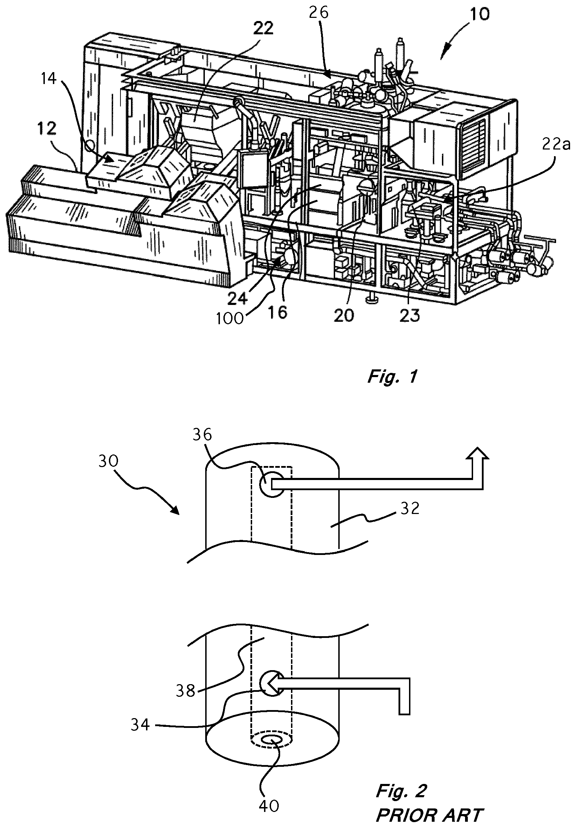

Referring now to Fig. 1 there is shown an exemplary form, fill and seal packaging machine 10 embodying the principles of the present disclosure. A conventional form, fill and seal packaging machine 10 includes a carton magazine 12 for storing flat, folded, carton blanks, a carton erection station 14 and a bottom forming and sealing station 22. The machine 10 further includes a sterilization station 16 for sterilizing the cartons and further includes a filling station 20 at which the cartons are filled with product and a top sealing station 22a at which the top panels of the cartons are pre-folded and subsequently sealed to one another. The cartons are then off loaded from the form, fill and seal packaging machine 10.

The sterilization station 16 is positioned between the bottom forming and sealing station 22 and the filling station 20. The sterilization station 16 can include one or more ultraviolet energy generating devices 24, and a hydrogen peroxide vapor generating system 26. The hydrogen peroxide vapor generating system 26 includes a hydrogen peroxide evaporation device 100.

Before turning into details of the various examples of hydrogen peroxide evaporation devices 100, a prior art evaporator 30 will be briefly described with reference to Fig. 2. The evaporator 30 has a cylindrical housing 32 having two closed ends. The housing 32 has an inlet 34 for receiving liquid hydrogen peroxide and an outlet 36 for discharging gaseous hydrogen peroxide. Inside the housing an electrical heating element 38 is arranged, extending along the longitudinal axis of the housing 32. The electrical heating element 38 has an electrical contact 40 extending on the outer side of the closed end of the housing 32 for connecting to a power supply (not shown). During operation liquid hydrogen peroxide is injected through the inlet 34, hitting against electrical heating element 38 whereby evaporation immediately occurs. Typically, for a desired outlet temperature of 200-250°C the surface temperature of the heating element 38 is approximately 600°C. As the gas is transported towards the outlet 36 the temperature of the gas gradually rises to reach the desired outlet temperature upon exit through the outlet 36. Due to the extremely high surface temperature of the heating element 38 corrosion will occur, and hence an increased breakdown rate of the hydrogen peroxide. Hence, it is not possible to accurately control the actual hydrogen peroxide concentration of the gas exiting the evaporator.

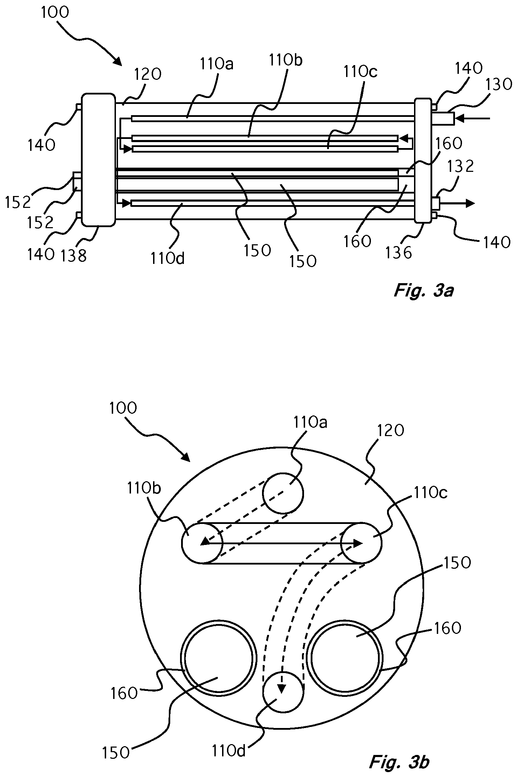

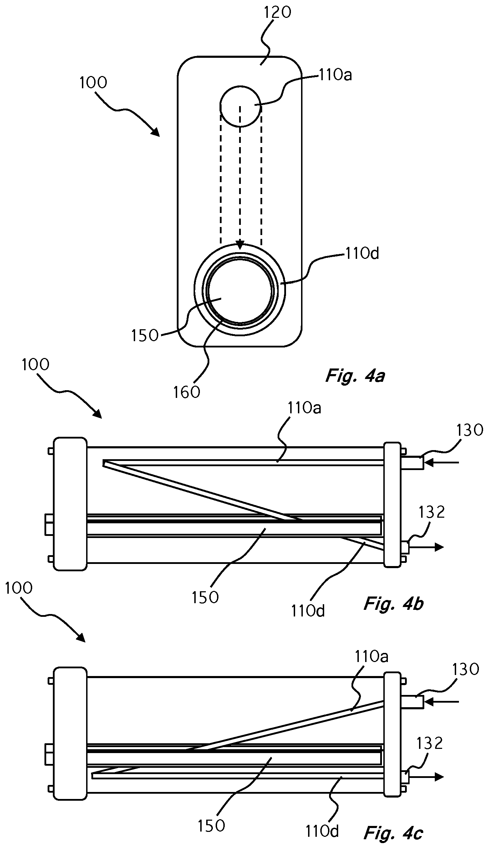

Examples of the evaporation device 100 will from hereon be described with reference to Figs. 3a-b and 4a-b. In this particular context the evaporation device 100 is used for evaporating hydrogen peroxide, however the evaporation device 100 may also be used for evaporating other liquids.

The hydrogen peroxide evaporation device 100 is not exclusively intended for the machine type described with reference to Fig. 1, but for all fill machines utilizing hydrogen peroxide vapor for sterilizing ready-to-fill packages.

In Fig. 3a and 3b a first example of a hydrogen peroxide evaporation device 100 is shown. The device 100 comprises a plurality of fluid channels 110a-d provided inside a housing body 120. The housing body 120 is preferably a solid block of metal, such as Aluminum, wherein the fluid channels 110a-d are drilled channels within the solid block 120. The drilled channels 110a-d are connected to each other such that they form a common fluid line. In the shown example, one end of the first fluid channel 110a is connected to one end of the second fluid channel 110b, wherein the opposite end of the second fluid channel 110b is connected to one end of the third fluid channel 110c. The opposite end of the third fluid channel 110c is connected to one end of the fourth fluid channel 110d. The first fluid channel 110a is connected to a fluid inlet 130 at the end being opposite the end connecting to the second fluid channel 110b. In a similar manner the fourth fluid channel 110d is connected to a fluid outlet 132 at the end being opposite the end connecting to the third fluid channel 110c.

The inlet 130 and the outlet 132 are preferably arranged on an end plate 136 sealing off the end face of the housing body 120. Moreover connection means may be provided at the inlet 130 and the outlet 132, respectively for allowing hoses or similar to be securely attached to the device 100. The connections between the fluid channels 110a-d are shown only schematically be arrows in Fig. 3a. However, these connections may also be drilled channels. For manufacturing facilitation the connections between the fluid channels 110a-d may be provided as grooves at the end faces of the housing body 120, whereby the previously mentioned end plate 136 will seal off the groove between the second and third fluid channel 110b,c such that it forms a fluid channel.

In a similar manner an end plate 138 may be provided at the opposite end face of the housing body 120 such that the fluid connection between the first and second fluid channel 110a,b and the fluid connection between the third and fourth fluid channel 110c,d may be provided as grooves at the end face of the housing body 120, whereby the end plate 138 will seal off the groove between the first and second fluid channel 110a,b and the groove between the third and fourth fluid channel 110c,d such that they form fluid channels.

Screws 140 may be used to secure the end plates 136, 138 to the housing body 120.

As can be seen in Fig. 3a the housing body 120 has a longitudinal extension, and the fluid channels 110a-d extend substantially in parallel with the longitudinal axis of the housing body 120.

The device 100 further comprises at least one electrical heating element 150, such as a heating cartridge or similar. Each heating element 150 is arranged in a tubular cavity 160 provided in the housing body 120. The tubular cavity 160 is preferably extending from one end face to the other, such that it forms a through hole along the longitudinal axis of the housing body 120. The end plate 138 is provided with electrical connections 152 for connecting the electrical heating element(s) 150 to a power supply (not shown).

In the shown example to tubular cavities 160 are provided, each cavity 160 enclosing an electrical heating element 150. The heating elements 150 extend in parallel with each other and with the longitudinal axis of the housing body 120. The length of each heating element 150 is preferably only slightly less than the total length of the housing body 120.

During operation the electrical heating elements 150 will be turned on, resulting in heating of the inner walls of the cavities 160. The temperature of the housing body 120 will thus gradually increase during operation. Due to the configuration of the heating elements 150 the temperature profile within the housing body 120 will be subject to a gradient whereby the temperature of the housing body 120 will be lower as the radial distance from the heating elements 150 increase.

In Fig. 3b the fluid channels 110a-d are seen from the end plate 136. The heating elements 150, arranged inside the tubular cavities 160 provide heating of the housing body 120. Hence, the temperature of the housing body 120 will be highest in the close proximity of the cavities 160.

Liquid aqueous solution of hydrogen peroxide, typically at a concentration of 2-5%, is fed into the first fluid channel 110a. As the liquid is flowing through this channel 110a it will be exposed to heat from the inner walls of the channel 110a. For optimal performance it is desired to keep the temperature of the first fluid channel 110a such that heat transfer is maximized. In accordance with the theories of heat transfer, maximum efficiency is obtained if the temperature of the fluid channel walls is approximately 130°C for diluted hydrogen peroxide. That is, the temperature of the inner wall of the fluid channel 110a should be approximately 30°C above the boiling temperature of the liquid to be heated. At specific fluid flows it has been shown that this temperature is actually sufficient for providing complete boiling of the liquid hydrogen peroxide. For having this temperature at the upper part of the housing body 120 it has been shown that the heating elements 150 should be heated to approximately 300°C, which is around half the temperature of the prior art solution described with reference to Fig. 2.

When the boiled hydrogen peroxide reaches the end of the first fluid channel 110a it will flow further into the second fluid channel 110b, and subsequently into the third fluid channel 110c. While passing the second and third fluid passage 110b, c, the temperature of the gas will increase gradually. Final heating of the gas is provided when the gas flows into the fourth fluid channel 110d, which is arranged in close proximity to the cavities 160. While passing through the fourth fluid channel 110d the gas will obtain its desired outlet temperature, which normally is within 200-250°C.

For the hydrogen peroxide evaporation device 100 the maximum temperature can be reduced by approximately 50% compared to prior art. This is partly due to the fact that maximum heat transfer for evaporation, and a gradual temperature increase is thereafter accomplished. Due to the reduction of the maximum temperature of the heating elements 150 corrosion is greatly reduced, as well as chemical breakdown of the hydrogen peroxide. For this reason it will be much easier to ensure the correct concentration of the discharged gas.

In Fig. 4a another example of a hydrogen peroxide evaporation device 100 is shown. Also in this example the housing body 120 is a solid block having drilled fluid channels 110a, 110d. A tubular cavity 160 is also provided for receiving a heating element 150 in the same manner as the example described with reference to Figs. 3a-b. The first fluid channel 110a is a longitudinal channel extending from an inlet and into the housing body 120 in the same manner as for the previous example. The first fluid channel 110a is connected to the second and final fluid channel 110d which is formed as an annular conduit coaxially around the cavity 160. Due to the arrangement of the fluid channels 110a, d relative the heating element 150 the gradient temperature profile within the housing body 120 will be obtained in a similar manner as for the previous example.

In Fig. 4b a yet further example of a hydrogen peroxide evaporation device 100 is shown. In this example two fluid channels 110a, d form the entire conduit for hydrogen peroxide inside the housing body 120. While the first fluid channel 100a extends in a similar manner as for the examples previously described, the second fluid channel 110d is tilted downwards for having one end connecting with the end of the first fluid channel 110a, and the opposite end connecting with the outlet 132. The heating elements 150 are identical to the heating elements 150 of Figs. 3a-b.

Fig. 4c shows a yet further example of a hydrogen peroxide evaporation device 100. The device 100 is almost identical to the device 100 of Fig. 4b, except for that the first fluid channel 110a is tilted while the second fluid channel 110d is parallel with the longitudinal extension of the housing body 120 and the heating element(s) 150.

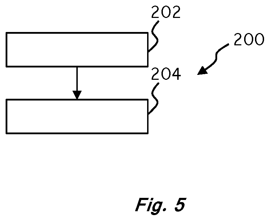

In Fig. 5 a method 200 for evaporating hydrogen peroxide is schematically shown. The method 200 comprises a first step 202 offeeding a liquid aqueous solution of hydrogen peroxide through a first fluid channel 110a arranged in a housing body 120, and subsequently through a second fluid channel 110d also arranged within said housing body 120. As described previously, the fluid channels 110a-d are connected to each other to form a common fluid line between an inlet 130 and an outlet 132. The method further comprises a subsequent step 204 of heating the inner walls of said fluid channels 110a-d by means of at least one heating element 150 arranged within said housing body 120. Step 204 is performed such that the first fluid channel 110a, being directly connected to the fluid inlet 130 and positioned relative the at least one heating element 150, will have its inner walls to be heated to a first temperature, and such that the second fluid channel 110d, being directly connected to the fluid outlet 132 and being positioned relative the at least one heating element 150, will have its inner walls heated to a second temperature, said second temperature being higher than the first temperature.

As described above, the method 200 is preferably performed such that the first temperature is between 120-140°C, and the second temperature is between 200-250°C.

The described examples are particularly suitable for applications using hydrogen peroxide. A typical example involves the use of a concentration of the liquid aqueous solution of hydrogen peroxide of about 2-5%, however in other examples the concentration may be up to 35-40%.

Further, step 204 is preferably performed such that liquid aqueous solution of hydrogen peroxide entering the first fluid channel 110a will be entirely evaporated while flowing through the first fluid channel 110a.

The described device and method is particularly used for all kinds of liquids which are to be evaporated and heated to a temperature exceeding the evaporation temperature. According to some examples, the temperature of the first fluid channel 110a is selected to about 30°C above the boiling point of the liquid used.

## Claims

1. An evaporation device (100) for evaporating hydrogen peroxide, comprising
a housing body (120) having at least two fluid channels (110a-d) arranged therein, which fluid channels (110a-d) are connected to each other to form a common fluid line between an inlet (130) and an outlet (132), and
at least one heating element (150) positioned within said housing body (120) for heating said fluid channels (110a-d), wherein
a first fluid channel (110a), being directly connected to the fluid inlet (130), is positioned relative the at least one heating element (150) such that its inner walls will be heated to a first temperature,
**characterized in that**
a second fluid channel (110d), being directly connected to the fluid outlet (132), is positioned relative the at least one heating element (150) such that its inner walls will be heated to a second temperature, said second temperature being higher than the first temperature.

2. The device (100) according to claim 1, wherein the housing body (120) is a solid block and wherein the fluid channels (110a-d) are channels provided inside said block.

3. The device (100) according to claim 1 or 2, wherein the housing body (120) is made of Aluminum or stainless steel.

4. The device (100) according to any one of the preceding claims, wherein said at least one heating element (150) extends along a longitudinal axis of said housing body (120).

5. The device (100) according to any one of the preceding claims, wherein the at least one heating element (150) is an electrical heating element.

6. The device (100) according to any one of the preceding claims, wherein each fluid channel (110a-d) extends from a first end face of the housing body (120) to an opposite end of the housing body (120), and wherein each end face of the housing body (120) is closed by means of a respective end plate (136, 138).

7. The device (100) according to claim 6, wherein at least one fluid channel (110a-d) is connected to an adjacent fluid channel (110a-d) by means of a fluid connection formed as a groove in one of said end faces.

8. The device (100) according to claim 7, wherein said at least one groove is closed by means of one of said end plates (136, 138).

9. A method for evaporating hydrogen peroxide, comprising:
feeding a liquid aqueous solution of hydrogen peroxide through a first fluid channel (110a) arranged in a housing body (120), and subsequently through a second fluid channel (110d) also arranged within said housing body (120), which fluid channels (110a-d) are connected to each other to form a common fluid line between an inlet (130) and an outlet (132), and
heating the inner walls of said fluid channels (110a-d) by means of at least one heating element (150) arranged within said housing body (120), whereby
the first fluid channel (110a), being directly connected to the fluid inlet (130), is positioned relative the at least one heating element (150) such that its inner walls will be heated to a first temperature,
**characterized in that**
the second fluid channel (110d), being directly connected to the fluid outlet (132), is positioned relative the at least one heating element (150) such that its inner walls will be heated to a second temperature, said second temperature being higher than the first temperature.

10. Method according to claim 9, wherein the first temperature is between 120-140°C, and wherein the second temperature is between 200-250°C

11. The method according to claim 9, wherein the first temperature is 30°C above the boiling temperature of the liquid to be evaporated, and wherein the second temperature is between 200-250°C.

12. The method according to any on of claims 9-11, wherein the concentration of the liquid aqueous solution of hydrogen peroxide is between 2-5%.

13. The method according to any one of claims 9-12, wherein the first temperature is selected such that liquid aqueous solution of hydrogen peroxide entering the first fluid channel (110a) will be entirely evaporated while flowing through the first fluid channel (110a).

## Patentansprüche

1. Verdampfungsvorrichtung (100) zur Verdampfung von Wasserstoffperoxid, die Folgendes umfasst:
einen Gehäusekörper (120) mit mindestens zwei darin angeordneten Fluidkanälen (110a-d), wobei die Fluidkanäle (110a-d) miteinander verbunden sind, um eine gemeinsame Fluidleitung zwischen einem Einlass (130) und einem Auslass (132) zu bilden, und
zumindest ein Heizelement (150), positioniert innerhalb des Gehäusekörpers (120) zum Erhitzen der Fluidkanäle (110a-d), wobei
ein erster Fluidkanal (110a), der direkt mit dem Fluideinlass (130) verbunden ist, relativ zu dem zumindest einen Heizelement (150) so positioniert ist, dass seine Innenwände auf eine erste Temperatur erhitzt werden,
**dadurch gekennzeichnet, dass**
ein zweiter Fluidkanal (110d), der direkt mit dem Fluidauslass (132) verbunden ist, relativ zu dem zumindest einen Heizelement (150) so positioniert ist, dass seine Innenwände auf eine zweite Temperatur erhitzt werden, wobei die zweite Temperatur höher als die erste Temperatur ist.

2. Vorrichtung (100) nach Anspruch 1, wobei der Gehäusekörper (120) ein durchgehender Block ist und wobei die Fluidkanäle (110a-d) Kanäle sind, die innerhalb des Blockes bereitgestellt sind.

3. Vorrichtung (100) nach Anspruch 1 oder 2, wobei der Gehäusekörper (120) aus Aluminium oder Edelstahl gefertigt ist.

4. Vorrichtung (100) nach einem der vorhergehenden Ansprüche, wobei sich das zumindest eine Heizelement (150) entlang einer Längsachse des Gehäusekörpers (120) erstreckt.

5. Vorrichtung (100) nach einem der vorhergehenden Ansprüche, wobei das zumindest eine Heizelement (150) ein elektrisches Heizelement ist.

6. Vorrichtung (100) nach einem der vorhergehenden Ansprüche, wobei sich jeder Fluidkanal (110a-d) von einer ersten Endfläche des Gehäusekörpers (120) zu einem entgegengesetzten Ende des Gehäusekörpers (120) erstreckt, und wobei jede Endfläche des Gehäusekörpers (120) mittels einer entsprechenden Endplatte (136, 138) verschlossen ist.

7. Vorrichtung (100) nach Anspruch 6, wobei zumindest ein Fluidkanal (110a-d) mittels einer Fluidverbindung, die als eine Nut in einer der Endflächen ausgebildet ist, mit einem angrenzenden Fluidkanal (110a-d) verbunden ist.

8. Vorrichtung (100) nach Anspruch 7, wobei die zumindest eine Nut mittels einer der Endplatten (136, 138) verschlossen ist.

9. Verfahren zur Verdampfung von Wasserstoffperoxid, das Folgendes umfasst:
Zuführen einer flüssigen wässrigen Lösung von Wasserstoffperoxid durch einen ersten Fluidkanal (110a), angeordnet in einem Gehäusekörper (120), und nachfolgend durch einen zweiten Fluidkanal (110d), ebenfalls in dem Gehäusekörper (120) angeordnet, wobei die Fluidkanäle (110a-d) miteinander verbunden sind, um eine gemeinsame Fluidleitung zwischen einem Einlass (130) und einem Auslass (132) zu bilden, und
Erhitzen der Innenwände der Fluidkanäle (110a-d) mittels zumindest eines Heizelements (150), angeordnet in dem Gehäusekörper (120), wobei
der erste Fluidkanal (110a), der direkt mit dem Fluideinlass (130) verbunden ist, relativ zu dem zumindest einen Heizelement (150) so positioniert ist, dass seine Innenwände auf eine erste Temperatur erhitzt werden,
**dadurch gekennzeichnet, dass**
der zweite Fluidkanal (110d), der direkt mit dem Fluidauslass (132) verbunden ist, relativ zu dem zumindest einen Heizelement (150) so positioniert ist, dass seine Innenwände auf eine zweite Temperatur erhitzt werden, wobei die zweite Temperatur höher als die erste Temperatur ist.

10. Verfahren nach Anspruch 9, wobei die erste Temperatur zwischen 120-140 °C ist, und wobei die zweite Temperatur zwischen 200-250 °C ist.

11. Verfahren nach Anspruch 9, wobei die erste Temperatur 30 °C über der Siedetemperatur der zu verdampfenden Flüssigkeit liegt und wobei die zweite Temperatur zwischen 200-250 °C ist.

12. Verfahren nach einem der Ansprüche 9-11, wobei die Konzentration der flüssigen wässrigen Lösung von Wasserstoffperoxid zwischen 2-5 % ist.

13. Verfahren nach einem der Ansprüche 9-12, wobei die erste Temperatur so ausgewählt wird, dass die flüssige wässrige Lösung von Wasserstoffperoxid, die in den ersten Fluidkanal (110a) eintritt, vollständig verdampft wird, während sie durch den ersten Fluidkanal (110a) strömt.

## Revendications

1. Dispositif d'évaporation (100) pour évaporer du peroxyde d'hydrogène, comprenant :
un corps de boîtier (120) ayant au moins deux voies de fluide (110a-d) disposées à l'intérieur de celui-ci, ces voies de fluide (110a-d) étant raccordées l'une à l'autre afin de former une conduite de fluide commune entre une entrée (130) et une sortie (132), et
au moins un élément de chauffage (150) positionné à l'intérieur dudit corps de boîtier (120) pour chauffer lesdites voies de fluide (110a-d), dans lequel
une première voie de fluide (110a), étant raccordée directement à l'entrée de fluide (130), est positionnée par rapport à l'au moins un élément de chauffage (150) de manière à ce que ses parois internes soient chauffées jusqu'à une première température, **caractérisé en ce que**
une deuxième voie de fluide (110d), étant raccordée directement à la sortie de fluide (132), est positionnée par rapport à l'au moins un élément de chauffage (150) de manière à ce que ses parois internes soient chauffées jusqu'à une deuxième température, ladite deuxième température étant plus élevée que la première température.

2. Dispositif (100) selon la revendication 1, dans lequel le corps de boîtier (120) est un bloc massif et dans lequel les voies de fluide (110a-d) sont des voies prévues à l'intérieur dudit bloc.

3. Dispositif (100) selon la revendication 1 ou 2, dans lequel le corps de boîtier (120) est fait en aluminium ou en acier inoxydable.

4. Dispositif (100) selon l'une quelconque des revendications précédentes, dans lequel ledit au moins un élément de chauffage (150) s'étend le long d'un axe longitudinal dudit corps de boîtier (120).

5. Dispositif (100) selon l'une quelconque des revendications précédentes, dans lequel l'au moins un élément de chauffage (150) est un élément de chauffage électrique.

6. Dispositif (100) selon l'une quelconque des revendications précédentes, dans lequel chaque voie de fluide (110a-d) s'étend depuis une première face d'extrémité du corps de boîtier (120) jusqu'à une extrémité opposée du corps de boîtier (120), et dans lequel chaque face d'extrémité du corps de boîtier (120) est fermée au moyen d'une plaque d'extrémité respective (136, 138).

7. Dispositif (100) selon la revendication 6, dans lequel au moins une voie de fluide (110a-d) est raccordée à une voie de fluide adjacente (110a-d) au moyen d'un raccord de fluide formé comme une gorge sur une desdites faces d'extrémité.

8. Dispositif (100) selon la revendication 7, dans lequel ladite au moins une gorge est fermée au moyen d'une desdites plaques d'extrémité (136, 138).

9. Procédé pour évaporer du peroxyde d'hydrogène, comprenant :
l'alimentation d'une solution aqueuse liquide de peroxyde d'hydrogène à travers une première voie de fluide (110a) disposée dans un corps de boîtier (120), et ensuite à travers une deuxième voie de fluide (110d) disposée aussi à l'intérieur dudit corps de boîtier (120), ces voies de fluide (110a-d) étant raccordées l'une à l'autre afin de former une conduite de fluide commune entre une entrée (130) et une sortie (132), et
le chauffage des parois internes desdites voies de fluide (110a-d) au moyen d'au moins un élément de chauffage (150) disposé à l'intérieur dudit corps de boîtier (120), comme quoi
la première voie de fluide (110a), étant raccordée directement à l'entrée de fluide (130), est positionnée par rapport à l'au moins un élément de chauffage (150) de manière à ce que ses parois internes soient chauffées jusqu'à une première température,
**caractérisé en ce que**
la deuxième voie de fluide (110d), étant raccordée directement à la sortie de fluide (132), est positionnée par rapport à l'au moins un élément de chauffage (150) de manière à ce que ses parois internes soient chauffées jusqu'à une deuxième température, ladite deuxième température étant plus élevée que la première température.

10. Procédé selon la revendication 9, dans lequel la première température est située entre 120°C et 140°C, et dans lequel la deuxième température est située entre 200°C et 250°C.

11. Procédé selon la revendication 9, dans lequel la première température est 30°C au-dessus de la température d'ébullition du liquide à évaporer, et dans lequel la deuxième température est située entre 200°C et 250°C.

12. Procédé selon l'une quelconque des revendications 9 à 11, dans lequel la concentration de la solution aqueuse liquide de peroxyde d'hydrogène est entre 2 % et 5 %.

13. Procédé selon l'une quelconque des revendications 9 à 12, dans lequel la première température est sélectionnée de manière à ce que la solution aqueuse liquide de peroxyde d'hydrogène entrant dans la première voie de fluide (110a) soit entièrement évaporée tandis qu'elle s'écoule à travers la première voie de fluide (110a).
